# EUROPEAN PATENT APPLICATION

(11) **EP 4 524 232 A1**
(43) Date of publication of application: **19.03.2025**
(21) Application number: 24197984.8
(22) Date of filing: 02.09.2024
(51) Int. Cl.: C12M 3/00

(54) **CELL PRODUCTION CARTRIDGE AND SYSTEM**

(30) Priority: 04.09.2023 JP 2023143042
(71) Applicant: CANON KABUSHIKI KAISHA, Tokyo 146-8501 (JP); Canon Medical Systems Corporation, Tochigi 324-0036 (JP)
(72) Inventor: YAMAGUCHI, Yosuke, Otawara-shi, 324-0036 (JP); YANAGITA, Kazutaka, Tokyo, 146-8501 (JP); SAKAI, Tetsuya, Tokyo, 146-8501 (JP); KUBOYAMA, Akane, Tokyo, 146-8501 (JP)
(74) Representative: Marks & Clerk LLP

(57) **Abstract**

According to one embodiment, a cell production cartridge includes a flow channel and a container for accommodating the flow channel. The container includes an energy transmission member for receiving, from outside the container, energy for sending a liquid within the flow channel. The energy transmission member is located at a boundary between an inside and an outside of the container.

## Description

### FIELD

Embodiments described herein relate generally to a cell production cartridge and a system.

### BACKGROUND

A cell production process for producing cells includes, for example, a step of extracting a cell from donor tissue and a step of culturing the extracted cell. These steps are performed using large-scale installations such as a centrifugal separator, in combination with manual operations by engineers. Arrangement and maintenance of large-scale installations are, however, very expensive. Also, the series of steps involve complicated and delicate work, and a difference in personal skills in the manual operations could incur variations in qualities of resultant cells.

Thus, in order to produce high-quality cells at low cost, it is necessary to automate the cell production process by an apparatus. Automation by an apparatus may, however, incur a risk of creating an unexpected pressure to send a cell solution through a flow channel piping, which could loosen the piping and cause leakage of the liquid. As such, constructing an environment that secures safety, for example, a safety cabinet or a laboratory which complies with biosafety level 2 (BSL-2), is required.

However, a safety cabinet, a BSL-2 compliant environment, or the like necessitates a large investment for facilities in addition to the apparatus, and this makes it difficult to manufacture inexpensively. Therefore, there is a demand for securing safety without necessitating a facility investment associated with the risk of liquid leakage.

### SUMMARY

In relation to the embodiments, the following disclosures are additionally given, which set forth some of the various aspects and optional features of the inventions.
(1) A cell production cartridge including a flow channel and a container for accommodating the flow channel. The container may include an energy transmission member for receiving, from outside the container, energy for sending a liquid within the flow channel. The energy transmission member may be located at the boundary between the inside and the outside of the container.
(2) The flow channel may include a reservoir for storing a liquid for use in cell production. At least a part of the reservoir may be movable. The energy transmission member may transfer a driving force corresponding to the energy to this at least a part of the reservoir.
(3) The energy transmission member may be movable and may relay the energy to the reservoir in the form of a mechanical action.
(4) The reservoir may include a syringe.
(5) The energy transmission member may be a diaphragm.
(6) The energy transmission member may be a bellows.
(7) The energy transmission member may be a balloon.
(8) The container may include a container body in which a concave portion for holding the flow channel is formed, and a lid attached to the container body so as to cover the concave portion. The lid may include the energy transmission member at a position for covering the reservoir in the flow channel.
(9) A closing member may be further included. The closing member is held by the container body and the lid so as to be movable in a direction orthogonal to the flow channel, and closes the flow channel by elastically deforming a part of the flow channel.
(10) The flow channel may further include a tube structure for permitting the liquid discharged from the reservoir to flow through it, and a connector for detachably connecting the tube structure to the reservoir.
(11) The flow channel may be a closed-system flow channel.
(12) The container may be a closed-system container.
(13) A system including the cell production cartridge and a liquid sending apparatus for applying the energy to the energy transmission member.

### BRIEF DESCRIPTION OF THE DRAWINGS

FIG. 1 is a perspective view schematically showing one example of a cell production cartridge and its peripheral configurations according to an embodiment.
FIG. 2 is a perspective view schematically showing an appearance of the cell production cartridge shown in FIG. 1.
FIG. 3 is a sectional view based on the arrow III-III indicated in FIG. 2.
FIG. 4 is a schematic diagram for explaining functions of the cell production cartridge shown in FIG. 1.
FIG. 5 is a schematic diagram for explaining a diaphragm through which the cell production cartridge receives energy from a liquid sending mechanism shown in FIG. 1.
FIG. 6 is a schematic diagram for explaining a balloon through which the cell production cartridge receives energy from the liquid sending mechanism shown in FIG. 1.
FIG. 7 is a sectional view schematically showing a state in which a pin member of the cell production cartridge shown in FIG. 2 has opened a flow channel.
FIG. 8 is a sectional view schematically showing a state in which the pin member of the cell production cartridge shown in FIG. 2 has closed the flow channel.
FIG. 9 is a flowchart for explaining exemplary operations according to the embodiment.
FIG. 10 is a schematic diagram for explaining an exemplary operation according to the embodiment.
FIG. 11 is a schematic diagram for explaining an exemplary operation according to the embodiment.
FIG. 12 is a schematic diagram for explaining an exemplary operation according to the embodiment.
FIG. 13 is a schematic diagram for explaining an exemplary operation according to the embodiment.
FIG. 14 is a schematic diagram for explaining an exemplary operation according to the embodiment.
FIG. 15 is a schematic diagram for explaining a bellows which constitutes a modification of the embodiment.
FIG. 16 is a flowchart for explaining operations according to another modification of the embodiment.

### DETAILED DESCRIPTION

A cell production cartridge according to an embodiment will be described with reference to the drawings. By way of example, the description will assume instances where the cell production cartridge is applied to the production of induced pluripotent stem (iPS) cells. However, the cell production cartridge is not limited to the uses in the production of iPS cells, but is applicable to the production of any kinds or types of cells.

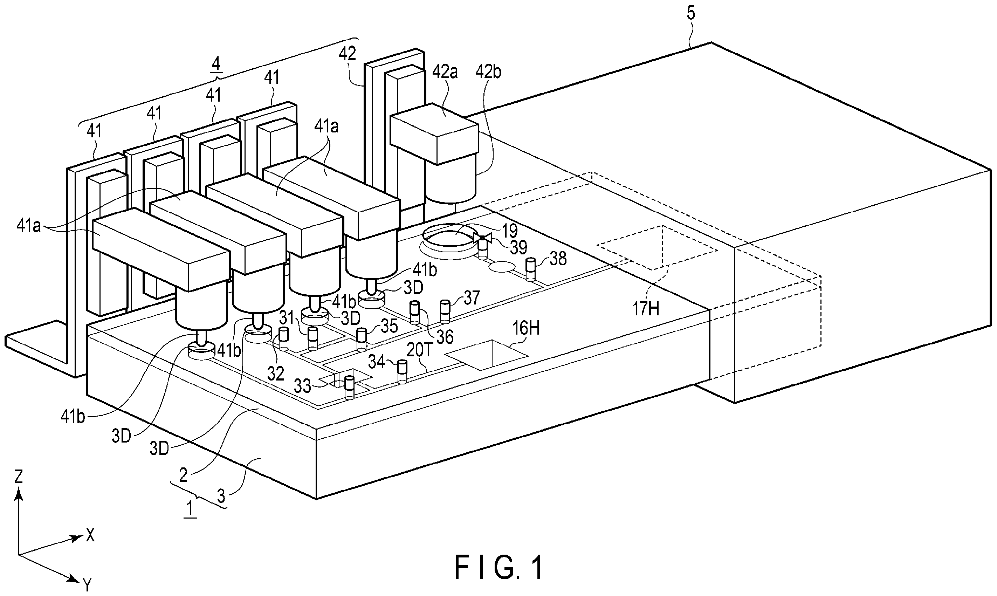

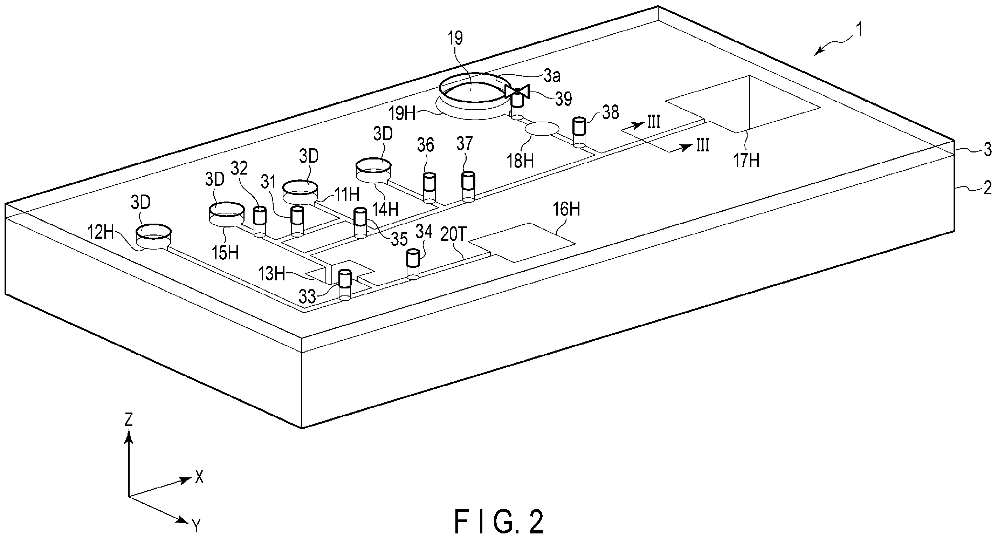

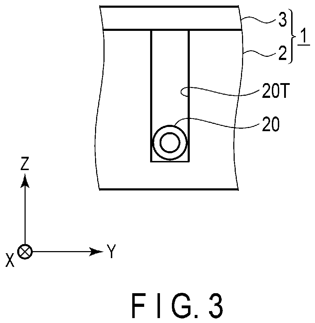

FIG. 1 is a perspective view schematically showing one example of the cell production cartridge and its peripheral configurations according to one embodiment, and FIG. 2 is a perspective view schematically showing the appearance of the cell production cartridge shown in FIG. 1. FIG. 3 is a sectional view based on the arrow III-III indicated in FIG. 2. In one example, the cell production cartridge, which is denoted by reference sign "1" here, is arranged near a liquid sending mechanism (liquid sending apparatus) 4 and detachably held by a culturing apparatus 5. The cell production cartridge 1 includes closed-system flow channels and a closed-system container which accommodates the flow channels. The closed-system flow channels have a first closed architecture formed by connecting a tube structure, reservoirs, etc., serving as paths for transporting a cell-containing liquid. The closed-system container has a second closed architecture outside the flow channels of the first closed architecture so that a liquid does not leak out from the cell production cartridge 1. The cell production cartridge 1 therefore has a double-closed architecture constituted by the first closed architecture and the second closed architecture. Note that the closed-system flow channels, if their sterile condition is securely maintained by a sterile filter or the like, may communicate with the outside of the flow channels or the closed-system container via the sterile filter, etc. Here, the sterile filter, etc. may preferably be hydrophobic. The container of the cell production cartridge 1 includes a container body 2 which is made of a resin or a plastic material and in which a concave portion for holding the flow channels is formed, and a lid 3 attached to the container body 2 so as to cover the concave portion. The container body 2 and the lid 3 are rigid. Being rigid here means having an elastic modulus equal to or higher than 10 times the elastic modulus of a later-described tube structure 20 or a soft portion such as a bag. In one example, the container body 2 and the lid 3 are made of a resin or plastic material. The resin material is, for example, polycarbonate (PC) or polystyrene (PS). Preferably, at least one of the container body 2 and the lid 3 has a light transmitting property. It is preferable that one or both of the container body 2 and the lid 3 be light transmitting because, for example, it allows for easy observation of the cell production process. FIG. 2 shows an exemplary appearance of the cell production cartridge 1 in which the container body 2 is opaque and the lid 3 is transparent. The concave portion of the container body 2 is constituted by holes 11H, 12H, 14H to 17H, and 19H for accommodating given ends of the closed-system flow channels, holes 13H and 18H for accommodating given halfway points of the closed-system flow channels, and trenches 20T for accommodating intermediate parts of the closed-system flow channels. The lid 3 includes a hole 3a arranged above the hole 19H of the concave portion of the container body 2 so as to expose a balloon 19 put in the hole 19H. The lid 3 includes diaphragms 3D at its positions covering the respective holes 11H, 12H, 14H, and 15H of the concave portion of the container body 2. In other words, the lid 3 includes the diaphragms 3D at positions where it covers the respective reservoirs in the flow channels. Each of the diaphragms 3D and the balloon 19 is an example of an energy transmission member for receiving, from outside the container, energy for sending and delivering a liquid within the closed-system flow channels. The diaphragms 3D and the balloon 19 are each located at the boundary between the inside and the outside of the container. The cell production cartridge 1 includes pin members 31 to 38 and a handle 39 which are each in the state of penetrating through the lid 3 and being slidably provided in the applicable part of the trenches 20T of the container body 2. Note that the number of holes such as the holes 11H to 19H and the layout of the concave portion that corresponds to the shapes of the trenches 20T may be discretionarily changed and adopted according to the cell production process. However, in instances where the liquid sending mechanism 4 and the culturing apparatus 5 are concurrently attached to the cell production cartridge 1 for use, the adopted layout preferably arranges the regions for the liquid sending mechanism 4 (e.g., the holes 11H, 12H, 14H, 15H, and 19H) and the regions for the culturing apparatus 5 (e.g., the hole 17H) away from each other so that interference is avoided. On the other hand, in instances where the liquid sending mechanism 4 and the culturing apparatus 5 are attached to the cell production cartridge 1 and used one at a time, a layout in which the regions for the liquid sending mechanism 4 (e.g., the holes 11H, 12H, 14H, 15H, and 19H) and the regions for the culturing apparatus 5 (e.g., the hole 17H) are close to each other may be adopted. Similarly, any layout may be adopted for the hole 3a and the diaphragms 3D of the lid 3 and the pin members 31 to 38 and the handle 39 of the cell production cartridge 1. Note also that the container of the cell production cartridge 1 may include a portion permitting communication between the inside and the outside of the container via a sterile filter, etc. Here, the sterile filter, etc. may preferably be hydrophobic.

The liquid sending mechanism 4 includes first mechanisms 41 for applying energy to the respective diaphragms 3D and a second mechanism 42 for applying energy to the balloon 19. The first mechanisms 41 each include a first arm 41a having its end above the corresponding diaphragm 3D and a rod 41b held and controlled at this end of the first arm 41a and adapted to push a piston via the diaphragm 3D. The number of the first mechanisms 41 is the same as the number of the diaphragms 3D and may be changed as appropriate according to the number of the diaphragms 3D. The second mechanism 42 includes a second arm 42a having its end above the balloon 19 and a column 42b held and controlled at this end of the second arm 42a and adapted to push the balloon 19. The second mechanisms 42 may be more than one, and its number is the same as the number of the balloon 19 and may be changed according to the number of the balloon 19.

The culturing apparatus 5 adjusts the environment in the cell production cartridge 1 which it detachably holds. More specifically, and in one example, the culturing apparatus 5 adjusts the environment such as temperature, moisture content, CO₂ content, etc., of a culture section (not shown in the figure) accommodated in the hole 17H of the held cell production cartridge 1.

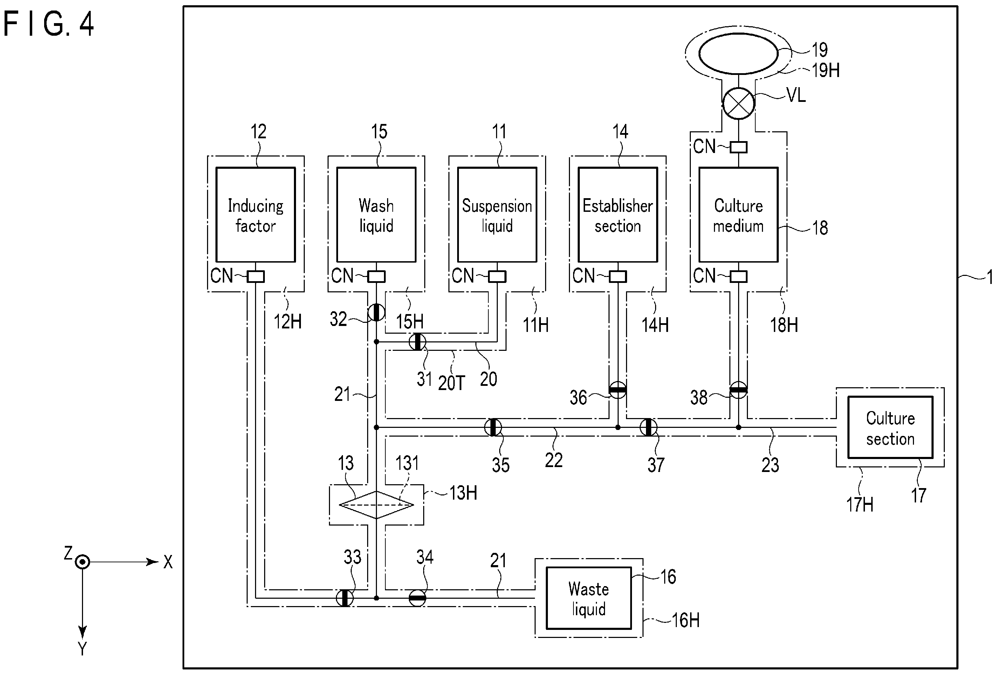

FIG. 4 is a schematic diagram for explaining functions of the cell production cartridge 1 shown in FIG. 1. The cell production cartridge 1, for constituting the closed-system flow channels, includes a suspension liquid feeder 11, an inducing factor feeder 12, a trap 13, an establisher section 14, a wash liquid feeder 15, a waste liquid receiver 16, a culture section 17, a culture medium feeder 18, the balloon 19, a tube structure 20, connectors CN, and a valve VL. The closed-system flow channels include the suspension liquid feeder 11, the inducing factor feeder 12, the establisher section 14, the wash liquid feeder 15, and the culture medium feeder 18 each as an at least partly movable reservoir for storing a liquid for use in the cell production. The diaphragms 3D each transfer a driving force corresponding to the energy to at least a part of the applicable reservoir (the suspension liquid feeder 11, the inducing factor feeder 12, the establisher section 14, or the wash liquid feeder 15). The balloon 19 transfers a driving force corresponding to the energy to at least a part of the applicable reservoir (the culture medium feeder 18). These diaphragms 3D and balloon 19 are movable members which relay the energy to the respective reservoirs in the form of a mechanical action. More specifically, portions of the cell production cartridge 1, i.e., the diaphragms 3D and the balloon 19, each have a configuration that can deform following the application of kinetic energy from the outside in order to transmit the liquid-sending energy to the inside of the closed-system flow channels. Similarly, each of the reservoirs at least partially has a configuration that can deform following the mechanical action transmitted from the corresponding energy transmission member. The suspension liquid feeder 11, the inducing factor feeder 12, the trap 13, the establisher section 14, the wash liquid feeder 15, the waste liquid receiver 16, the culture section 17, the culture medium feeder 18, the balloon 19, and the connectors CN are accommodated in the respective holes 11H to 19H of the concave portion. One or more supporting members (not shown in the figure) may be formed in or at the holes 11H to 19H to support the disposed objects such as a syringe or a bag. The tube structure 20 and the valve VL are accommodated in the trenches 20T. The trenches 20T may include fitting portions or joints to which the tube structure 20, the valve VL, the connectors CN, etc. can be fit. The tube structure 20 permits the liquids discharged from the respective reservoirs to flow through it. Each connector CN detachably couples the tube structure 20 with the corresponding reservoir.

In one example, the closed-system flow channels include a first flow channel 21, a second flow channel 22, and a third flow channel 23 which are formed of portions of the tube structure 20 and opened and closed by means of the pin members 31 to 38. As the components associated with the first flow channel 21, the wash liquid feeder 15, the suspension liquid feeder 11, the trap 13, the inducing factor feeder 12, and the waste liquid receiver 16 are arranged in this order in the flow direction (from the upstream side to the downstream side). The first flow channel 21 receives an inflow suspension liquid fed from the suspension liquid feeder 11 and an inflow inducing factor fed from the inducing factor feeder 12. In the first flow channel 21, the suspension liquid flows in a first direction, and the inducing factor flows in a second direction opposite the first direction. The second flow channel 22 is a channel branching off from the first flow channel 21 at a position upstream of the trap 13 and has the establisher section 14 downstream. Here, the position where the second flow channel 22 branches off is a position on the first flow channel 21 that is between the entrance for the suspension liquid and the trap 13. The third flow channel 23 is connected to the second flow channel 22 on the downstream side, and has the culture medium feeder 18 upstream and the culture section 17 downstream.

The suspension liquid feeder 11 feeds a suspension liquid containing target cells. The suspension liquid may be blood extracted from a human body in which the target cells are suspended, or a preservative solution in which the target cells are suspended. The target cells here refer to cells to be reprogrammed by the inducing factor. The target cells may be cells derived from blood or cells of other origin, as long as they are reprogrammable somatic cells. As the blood-derived target cells, mononuclear cells may be used.

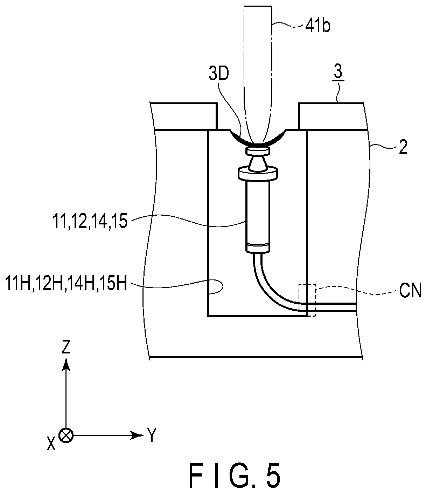

In one example, the suspension liquid feeder 11 is constituted by an injector including a syringe and a piston as shown in FIG. 5. The syringe is adapted to contain the suspension liquid with an airtight configuration so that the suspension liquid is inhibited or prevented from being exposed to the ambient air. In this example, the tip of the syringe is connected to the first flow channel 21 via the corresponding connector CN and also the portion of the tube structure 20 that has the pin member 31 located above. The piston is depressed by the corresponding rod 41b via the corresponding diaphragm 3D so as to push out the suspension liquid contained in the syringe. The syringe is an example of the reservoir.

The inducing factor feeder 12 feeds an inducing factor-containing culture medium to the trap 13. The inducing factor-containing culture medium is formed of an inducing factor and a culture medium for establishing iPS cells. The inducing factor is employed for reprogramming the target cells and includes, as concrete examples, one or more of Oct family genes, Klf family genes, Myc family genes, and their respective gene products. In one example, Oct3/4 is used as the Oct family gene, Klf4 is used as the Klf family gene, and c-Myc or L-Myc is used as the Myc family gene. The inducing factor may also include Sox family genes and/or gene products. As the Sox family gene, Sox2 may be used.

In one example, the inducing factor feeder 12 is constituted by an injector including a syringe and a piston. The syringe is adapted to contain the inducing factor-containing culture medium with an airtight configuration so that the inducing factor-containing culture medium is inhibited or prevented from being exposed to the ambient air. In this example, the tip of the syringe is connected to the first flow channel 21 via the corresponding connector CN and also the portions of the tube structure 20 that have the pin members 33 and 34 located above. The piston is depressed by the corresponding rod 41b via the corresponding diaphragm 3D so as to push out the inducing factor-containing culture medium contained in the syringe.

The trap 13 is arranged at a position on the first flow channel 21 that is between the suspension liquid feeder 11 and the inducing factor feeder 12. In other words, the trap 13 is arranged between the entrance for the suspension liquid and the entrance for the inducing factor, in the middle of the first flow channel 21. The trap 13 traps the target cells contained in the suspension liquid fed from the suspension liquid feeder 11. In one example, the trap 13 is constituted by a container including a filter 131 with meshes that can catch the target cells while permitting other substances having a smaller particle size than the target cells to pass through.

The establisher section 14 is connected to the second flow channel 22 including the portions of the tube structure 20 that have the pin members 35 and 36 located above, so that the target cells and the inducing factor are fed to the establisher section 14 through the second flow channel 22. The establisher section 14 establishes iPS cells by introducing the inducing factor that has been fed to the trap 13 into the target cells that have been trapped by the trap 13. The establisher section 14 sends the established iPS cells to the culture section 17 via the second flow channel 22 and also the third flow channel 23 including the portion of the tube structure 20 that has the pin member 37 located above.

In one example, the establisher section 14 is constituted by an injector including a syringe and a piston. The syringe is adapted to contain the inducing factor-containing culture medium that has been fed from the inducing factor feeder 12 and passed through the trap 13, and the target cells that have been trapped by the trap 13. The syringe has an airtight configuration so that the inducing factor-containing culture medium and the target cells are inhibited or prevented from being exposed to the ambient air. Also, this syringe is adapted to contain the iPS cells established by the introduction of the inducing factor into the target cells. In this example, the tip of the syringe is connected to the second flow channel 22 via the corresponding connector CN and also the portion of the tube structure 20 that has the pin member 36 located above. The piston is depressed by the corresponding rod 41b via the corresponding diaphragm 3D so as to push out the iPS cells contained in the syringe.

The wash liquid feeder 15 feeds a wash liquid to the first flow channel 21 so that the trap 13 is washed by the wash liquid. The wash liquid is, for example, a saline solution or the like, and a liquid that gives very little damage to the target cells may be adopted as the wash liquid.

In one example, the wash liquid feeder 15 is constituted by an injector including a syringe and a piston. The syringe is adapted to contain the wash liquid with an airtight configuration so that the wash liquid is inhibited or prevented from being exposed to the ambient air. In this example, the tip of the syringe is connected to the first flow channel 21 via the corresponding connector CN and also the portion of the tube structure 20 that has the pin member 32 located above. The piston is depressed by the corresponding rod 41b via the corresponding diaphragm 3D so as to push out the wash liquid contained in the syringe.

The waste liquid receiver 16 is a container for receiving a waste liquid. The waste liquid receiver 16 receives, as the waste liquid, the suspension liquid and the wash liquid that have passed through the trap 13. The waste liquid receiver 16 may be a flask, a bag, or any container that can receive and keep the waste liquid.

The culture section 17 cultures the iPS cells established by the establisher section 14. The culture section 17 may be a well plate, a flask, a dish, a bag, or any container that can be used to culture iPS cells.

The culture medium feeder 18 feeds a culture medium for culturing the iPS cells to the culture section 17 via the portion of the tube structure 20 that has the pin member 38 located above and the third flow channel 23 connected via this portion of the tube structure 20. The culture medium feeder 18 may be a container for storing a culture medium and examples of it include a bag.

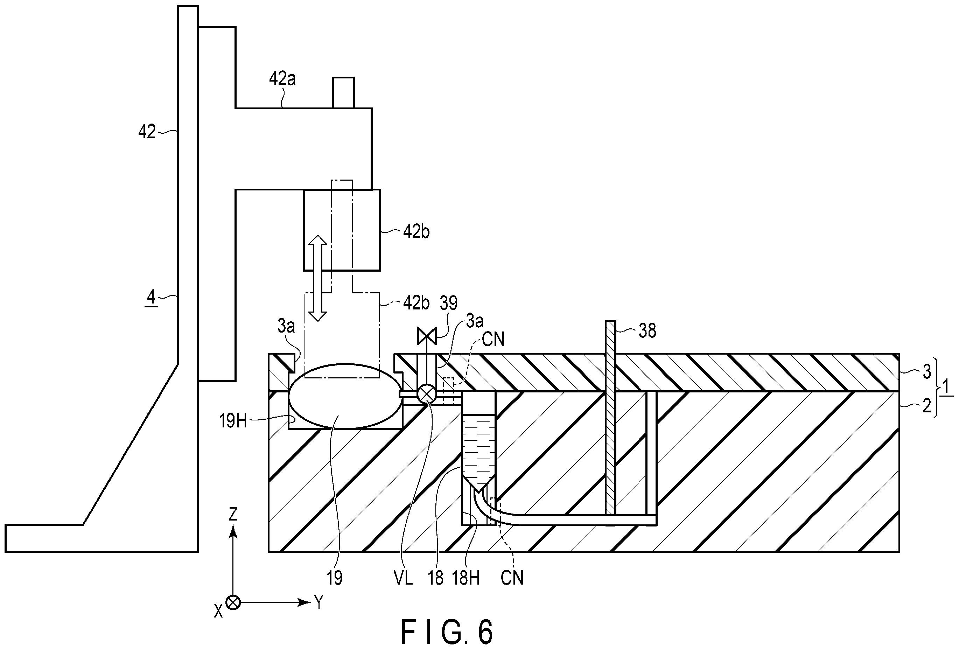

In one example, the culture medium feeder 18 is constituted by a bag with an upper end tube and a lower end tube as shown in FIG. 6. The bag is adapted to contain the culture medium with an airtight configuration so that the culture medium is inhibited or prevented from being exposed to the ambient air. The lower end tube of the bag is connected to the third flow channel 23 via the corresponding connector CN and the portion of the tube structure 20 that has the pin member 38 located above. The upper end tube of the bag is connected to the balloon 19 via the corresponding connector CN and the valve VL. The handle 39 is freely attachable to the valve VL and the valve VL is adapted to be opened and closed according to an operation of the attached handle 39. The balloon 19, upon being pressed by the column 42b, introduces the air contained in it into the upper part of the culture medium feeder 18 via the valve VL, the corresponding connector CN, and the upper end tube. The culture medium feeder 18 discharges the culture medium pressurized by the introduced air from the lower end tube to the third flow channel 23.

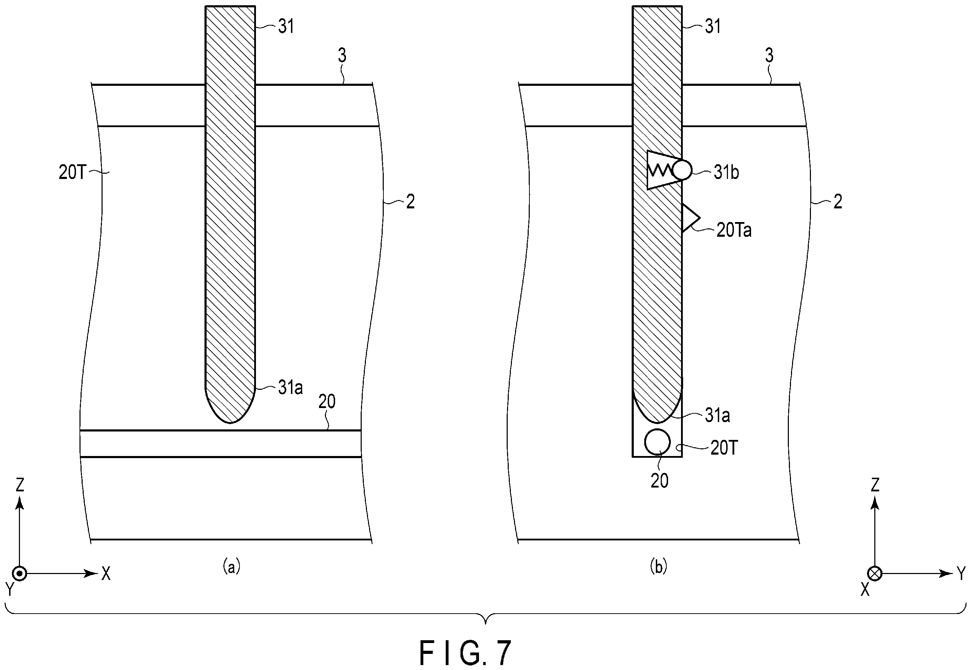

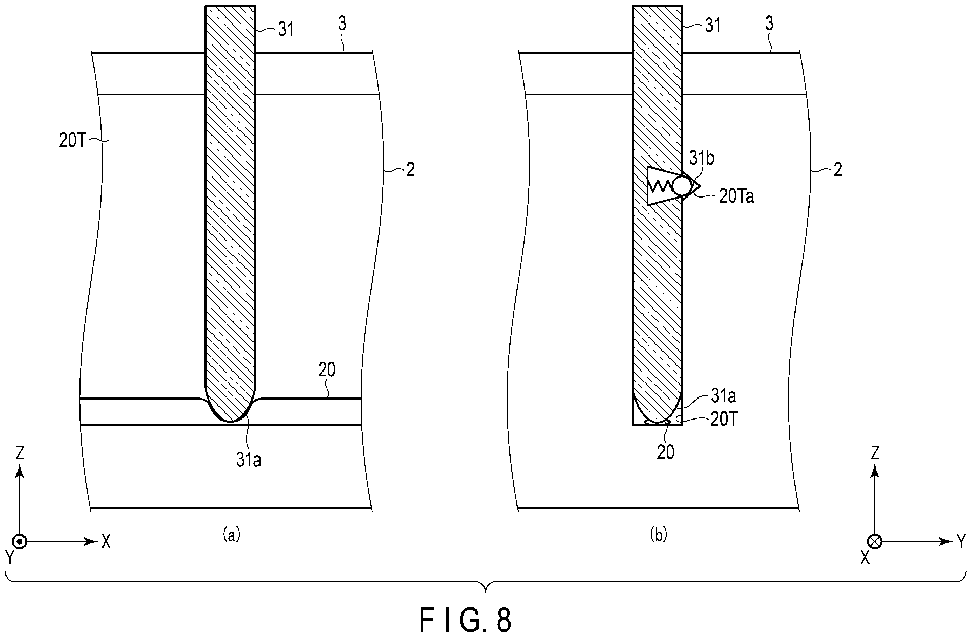

The portions of the tube structure 20 that constitute the first flow channel 21 are elastically deformable, and the pin members 31 to 34 are arranged above these portions in such a configuration that each of the pin members 31 to 34 can vertically ascend and descend. The pin members 31 and 32 are arranged above the respective portions of the tube structure 20 that are on one side (upstream side) of the first flow channel 21, and the suspension liquid feeder 11 and the wash liquid feeder 15 are connected to these portions. The pin member 31 switchably permits and stops the flow of the suspension liquid from the suspension liquid feeder 11 into the first flow channel 21 by switching between a state of opening the tube structure 20 as shown in FIG. 7 and a state of closing the tube structure 20 as shown in FIG. 8. Here, FIG. 7(a) shows a cross-section substantially parallel to the tube structure 20 and FIG. 7(b) shows a cross-section substantially orthogonal to the tube structure 20, in each of which a tip 31a of the pin member 31 is not in contact with the tube structure 20. Note that, as one example, the pin member 31 may be formed as a ball pin with an outwardly urged ball 31b. Such a ball 31b may function as a positioning member for positioning through engagement with a hole 20Ta formed in the trench 20T. Similarly, FIG. 8(a) shows a cross-section substantially parallel to the tube structure 20 and FIG. 8(b) shows a cross-section substantially orthogonal to the tube structure 20, in each of which the tip 31a of the pin member 31 closes the flow channel by squashing a part of the tube structure 20. The hole 20Ta in the trench 20T is formed so as to correspond to the position of the pin member 31 that closes the tube structure 20. Note that the other pin members 32 to 38 are adapted to function in the same manner as the pin member 31 as shown in FIGS. 7 and 8. The pin member 32 switchably permits and stops the flow of the wash liquid from the wash liquid feeder 15 into the first flow channel 21 by switching between a state of opening the tube structure 20 and a state of closing the tube structure 20. The pin member 33 is arranged above the portion of the tube structure 20 that is on the other side (downstream side) of the first flow channel 21, and the inducing factor feeder 12 is connected to this portion. The pin member 33 switchably permits and stops the flow of the inducing factor-containing culture medium into the first flow channel 21 by switching between a state of opening the tube structure 20 and a state of closing the tube structure 20. Also, in the middle of the first flow channel 21, the pin member 34 is arranged above the portion of the tube structure 20 that is connected to the inlet of the waste liquid receiver 16. The pin member 34 switchably permits and stops the flow of the suspension liquid and the wash liquid that have passed through the trap 13 into the waste liquid receiver 16 by switching between a state of opening the tube structure 20 and a state of closing the tube structure 20.

The pin members 35 to 37 are arranged above the portions of the tube structure 20 constituting the second flow channel 22. The pin member 35 switchably permits and stops the flow of the inducing factor-containing culture medium that has passed through the trap 13 and the target cells that have been trapped by the trap 13 into the second flow channel 22 by switching between a state of opening the tube structure 20 and a state of closing the tube structure 20. The pin members 36 and 37 switchably permit and stop the flow of the iPS cells established in the establisher section 14 into the third flow channel 23 by each switching between a state of opening the tube structure 20 and a state of closing the tube structure 20. The pin member 38 switchably permits and stops the flow of the culture medium from the culture medium feeder 18 into the third flow channel 23 by switching between a state of opening the tube structure 20 and a state of closing the tube structure 20. Each of the pin members 31 to 38 as described above is an example of a closing member held by the container body 2 and the lid 3 so as to be movable in a direction orthogonal to the flow channel and adapted to close the applicable flow channel by elastically deforming a part thereof.

The tube structure 20, which forms the first flow channel 21, the second flow channel 22, and the third flow channel 23, is constituted by, for example, airtight flexible tubular members such as vinyl or plastic tubes. Each liquid flowing through the first flow channel 21, the second flow channel 22, and the third flow channel 23 is thus inhibited or prevented from being exposed to the ambient air.

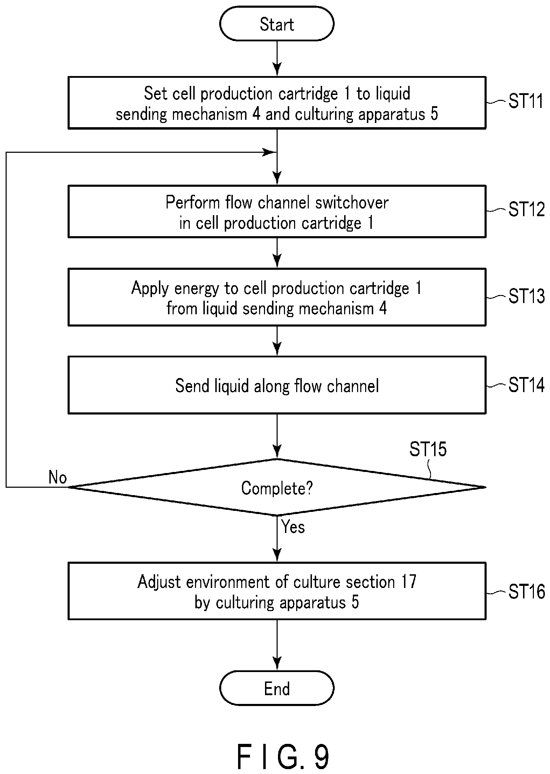

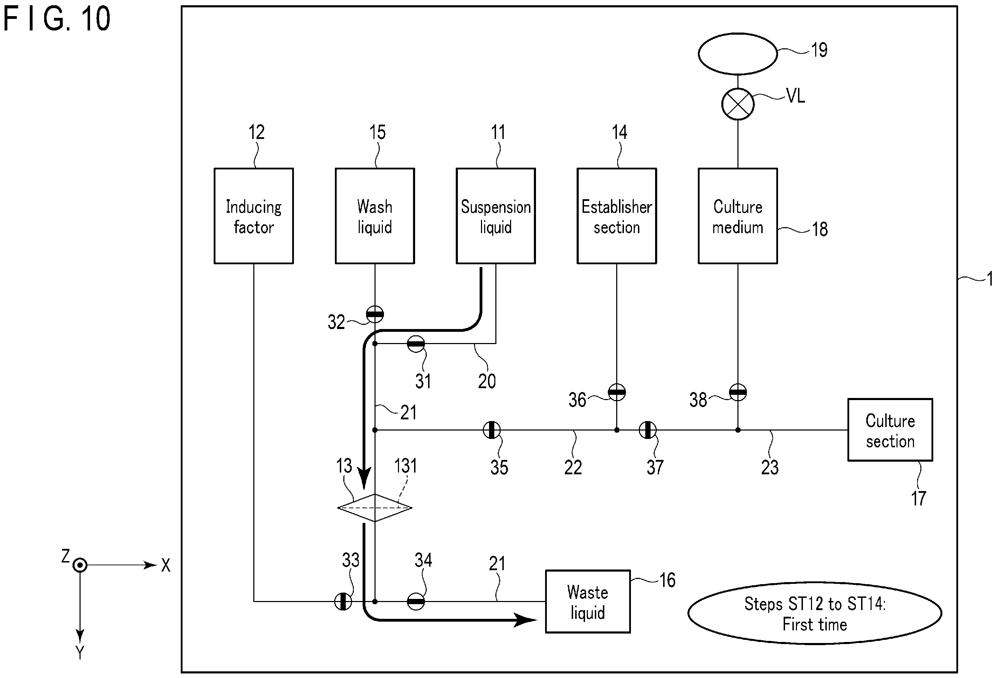

Exemplary operations conducted with the cell production cartridge and its peripheral configurations as explained above will be described using the flowchart in FIG. 9 and the schematic diagrams in FIGS. 10 to 14. The description will assume the suspension liquid to be blood collected from a donor. It will also be assumed that, in the cell production cartridge 1, the suspension liquid feeder 11 retains the blood, the inducing factor feeder 12 retains the inducing factor-containing culture medium, and the wash liquid feeder 15 retains the wash liquid, each in an amount corresponding to one process. That is, the cell production cartridge 1 here serves as a disposable product for use in one cell production process. It will be assumed that, in the cell production cartridge 1, the pin members 31 to 38 are in the state of closing the flow channels 21 to 23, and the valve VL is in its closed state. Note that the exemplary cell production process includes steps ST11 to ST16 which proceed by the action of an automatic mechanism constituted by the liquid sending mechanism 4, the culturing apparatus 5, a conveyor, a switcher, and a controller, without an intervention by a user. However, steps ST11 to ST16 may each involve user interventions as needed.

In step ST11, the cell production cartridge 1 is conveyed to a position below the liquid sending mechanism 4 by the conveyor (not shown in the figure) so as to be set in (attached to) the culturing apparatus 5. Subsequently, the switcher (not shown in the figure) capable of switching the states of the pin members 31 to 38 and operating the handle 39 is attached to the cell production cartridge 1.

In step ST12 after step ST11, the pin members 31 to 38 of the cell production cartridge 1 are each independently manipulated by the switcher (not shown in the figure) for their respective insertion levels so that the corresponding portions of the tube structure 20 are switched between the opened state and the closed state. This realizes the switchover among the flow channels 21 to 23.

In step ST13 after step ST12, the liquid sending mechanism 4 applies energy to the cell production cartridge 1. In one example, the applicable first mechanism 41 pushes the corresponding piston via the diaphragm 3D. Or, the second mechanism 42 pushes the balloon 19.

In step ST14 after step ST13, the cell production cartridge 1 sends a given liquid through the path after the switchover, according to the applied energy.

In step ST15 after step ST14, the controller (not shown in the figure) determines whether or not the cell production process has been completed. If the result of determination in this step is "No", the controller controls the switcher and the liquid sending mechanism 4 to return to step ST12 so that steps ST12 to ST15 are repeated. If, on the other hand, it is determined in step ST14 that the cell production process has been completed, the controller activates the culturing apparatus 5 to advance to step ST16.

In step ST16 after step ST15, the culturing apparatus 5 adjusts the environment of the culture section 17 accommodated in the cell production cartridge 1. Cells in the culture section 17 are thus cultured. The controller here controls the switcher and the liquid sending mechanism 4 as needed to supply a culture medium from the culture medium feeder 18 to the culture section 17 in the cell production cartridge 1. Then, upon finishing the culturing, the cell production process is ended.

As one example, a description will be given of, among the above described operations, how the operations in steps ST12 to ST14 are repeated for the flow channels in the cell production cartridge 1 and how the operation in step ST16 is performed.

### (Steps ST12 to ST14 for the first time)

In the cell production cartridge 1, the pin members 31 and 34 are manipulated by the switcher (not shown in the figure) so that their tip positions move upward to open the corresponding portions of the tube structure 20 as shown in FIG. 10. This causes a flow channel switchover in the cell production cartridge 1 by which the suspension liquid feeder 11 is placed in communication with the waste liquid receiver 16 via the trap 13 and the first flow channel 21 (step ST12). In FIG. 10, the pin members 31 to 38 are expressed by respective circle marks, and within each circle mark, the line overlapping the flow channel indicates an opened state of the flow channel and the line orthogonal to the flow channel indicates a closed state of the flow channel. The same applies to the other figures.

The liquid sending mechanism 4 applies energy to the suspension liquid feeder 11 by the applicable first mechanism 41 pushing the piston of the suspension liquid feeder 11 via the corresponding diaphragm 3D (step ST13).

According to the applied energy, the suspension liquid feeder 11 sends out the suspension liquid through the path after the switchover (step ST14). Here, the suspension liquid feeder 11 feeds the suspension liquid (blood) containing target cells in a forward direction through the first flow channel 21 to the trap 13. The forward direction is defined as a direction in which the suspension liquid flows toward the waste liquid receiver 16. More specifically, the suspension liquid feeder 11 discharges, to the first flow channel 21 and in the amount corresponding to one process, the suspension liquid which contains the target cells required for producing iPS cells. The suspension liquid discharged to the first flow channel 21 flows and enters the trap 13. Among the components (substances) contained in the suspension liquid flowing into the trap 13, the components having a particle size larger than the mesh opening of the filter 131 are caught, while the components having a particle size smaller than the mesh opening of the filter 131 are permitted to pass through. The filter 131 here may be a filter with meshes that can catch components each having, for example, a size equivalent to a white blood cell. Each target cell, having a particle size larger than the opening of such meshes, is caught by the filter 131. Red blood cells, etc., not handled as target cells in this example, each have a particle size smaller than the mesh opening, and therefore, they pass through the filter 131. The blood components that have passed through the trap 13 are, as a part of a waste liquid, received and kept by the waste liquid receiver 16.

### (Steps ST12 to ST14 for the second time)

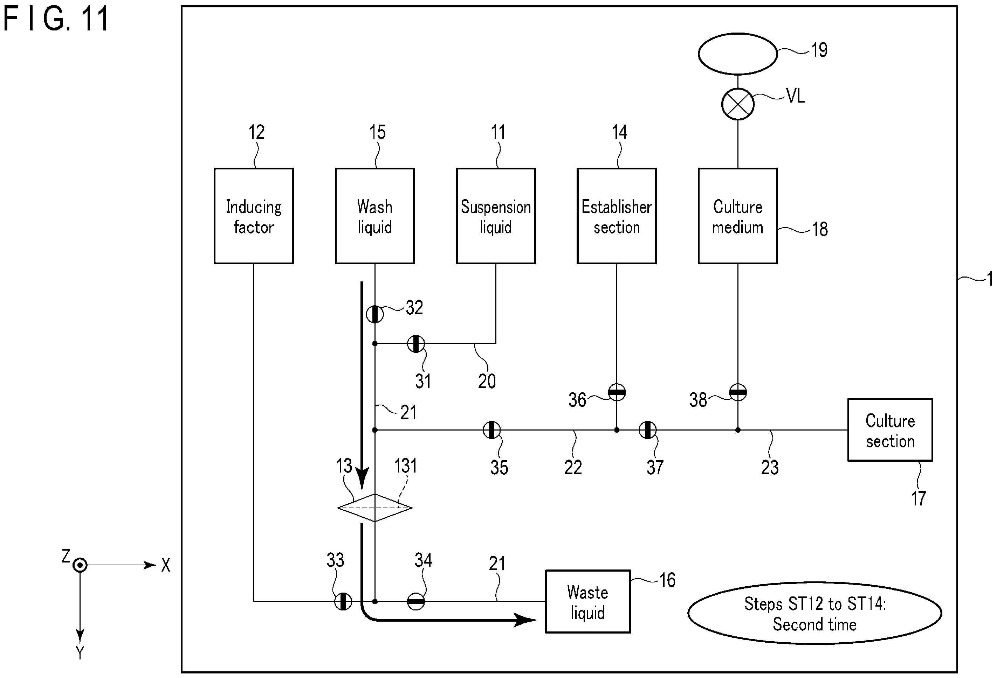

In the cell production cartridge 1, the pin members 31 and 32 are manipulated by the switcher (not shown in the figure) so that the tip position of the pin member 31 moves down to close the corresponding portion of the tube structure 20 and, subsequently, the tip position of the pin member 31 moves up to open the corresponding portion of the tube structure 20 as shown in FIG. 11. This causes a flow channel switchover in the cell production cartridge 1 by which the wash liquid feeder 15 is placed in communication with the waste liquid receiver 16 via the trap 13 and the first flow channel 21 (step ST12) .

The liquid sending mechanism 4 applies energy to the wash liquid feeder 15 by the applicable first mechanism 41 pushing the piston of the wash liquid feeder 15 via the corresponding diaphragm 3D (step ST13) .

According to the applied energy, the wash liquid feeder 15 sends out the wash liquid through the path after the switchover (step ST14). The wash liquid feeder 15 here causes the wash liquid to wash away the unwanted blood components remaining in the first flow channel 21 and the trap 13, other than the target cells, into the waste liquid receiver 16. In one example, components such as plasma, red blood cells, and platelets mainly constitute the unwanted blood components. Here, by adopting a configuration of advancing and retracting the piston of the wash liquid feeder 15 to repeat the discharge and suction of the wash liquid, it is possible to efficiently remove and sweep away the unwanted blood components sticking to the first flow channel 21 or the trap 13.

### (Steps ST12 to ST14 for the third time)

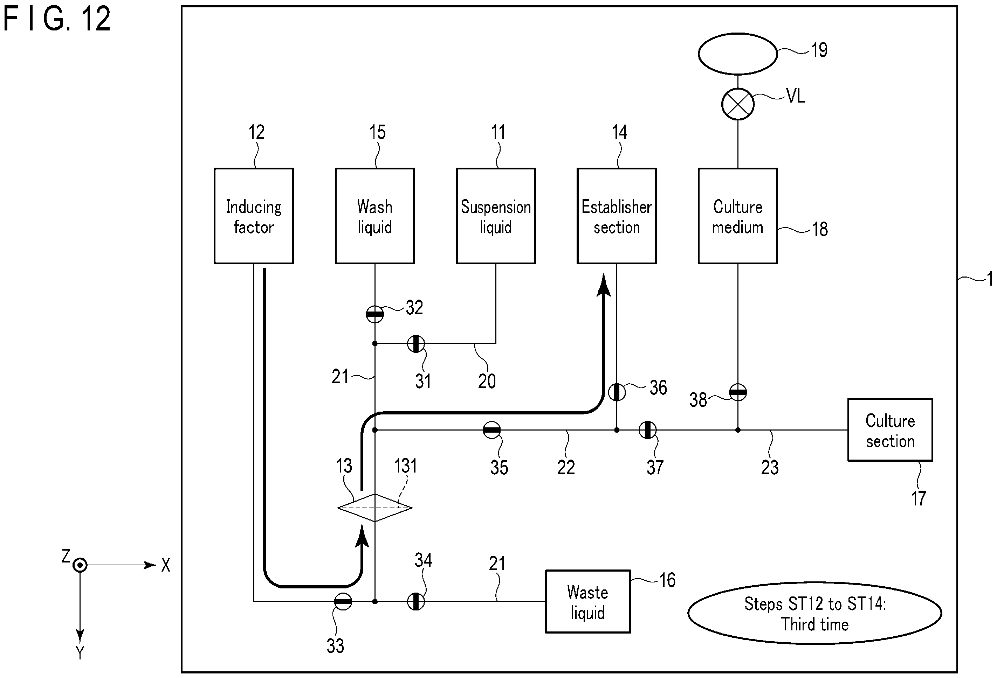

In the cell production cartridge 1, the pin members 32 to 36 are manipulated by the switcher (not shown in the figure) so that the tip positions of the respective pin members 32 and 34 move down to close the corresponding portions of the tube structure 20 and, subsequently, the tip positions of the respective pin members 33, 35, and 36 move up to open the corresponding portions of the tube structure 20 as shown in FIG. 12. This causes a flow channel switchover in the cell production cartridge 1 by which the inducing factor feeder 12 is placed in communication with the establisher section 14 via the trap 13 and the flow channels 21 and 22 (step ST12).

The liquid sending mechanism 4 applies energy to the inducing factor feeder 12 by the applicable first mechanism 41 pushing the piston of the inducing factor feeder 12 via the corresponding diaphragm 3D (step ST13).

According to the applied energy, the inducing factor feeder 12 sends out the inducing factor-containing culture medium through the path after the switchover (step ST14). The inducing factor feeder 12 here feeds the inducing factor-containing culture medium in the direction opposite the forward direction through the first flow channel 21 to the trap 13, so that the inducing factor-containing culture medium together with the target cells are fed to the establisher section 14 via the second flow channel 22. More specifically, the inducing factor feeder 12 discharges the retained one-process equivalent amount of the inducing factor-containing culture medium. The discharged inducing factor-containing culture medium flows into the trap 13 from the direction opposite the flow direction of the suspension liquid. The inducing factor-containing culture medium that has entered the trap 13 passes through the filter 131 and flows, together with the target cells caught by the filter 131, into the establisher section 14 via the second flow channel 22. Note that the piston of the establisher section 14 may be pulled to the limit beforehand in order to take in the inducing factor-containing culture medium and the target cells in the syringe of the establisher section 14.

In the establisher section 14, the inducing factor is introduced into the target cells and iPS cells are established from the target cells. The inducing factor may be provided in a variety of forms. For example, the inducing factor may be integrated into various types of vectors and provided. The vectors here are not limited, and viral vectors such as a Sendai virus vector and a retroviral vector, or non-viral vectors such as a plasmid may be suitably adopted. In an example where an inducing factor-integrated Sendai virus vector is used, the Sendai virus vector and the target cell are brought into contact with each other so that the inducing factor is introduced into the target cell. A gene product of the inducing factor is then generated. The gene product induces reprogramming of the target cell and thereby gives birth to an iPS cell which is an undifferentiated cell having a pluripotency and a proliferating ability. The thus-obtained iPS cells are left to grow in the presence of the culture medium for approximately an hour. In this manner, the iPS cells are established. During the establishing operation, the establisher section 14 may be suitably maintained at approximately 37 °C and 5% COz.

For the purpose of enhancing the accuracy of iPS cell establishment in the establisher section 14, a container of a relatively small capacity may be suitably used as the syringe of the establisher section 14. This can increase the likelihood of the target cells and the inducing factor or the inducing factor-integrated vector contacting each other, and consequently, can increase the likelihood of iPS cells being successfully established.

### (Steps ST12 to ST14 for the fourth time)

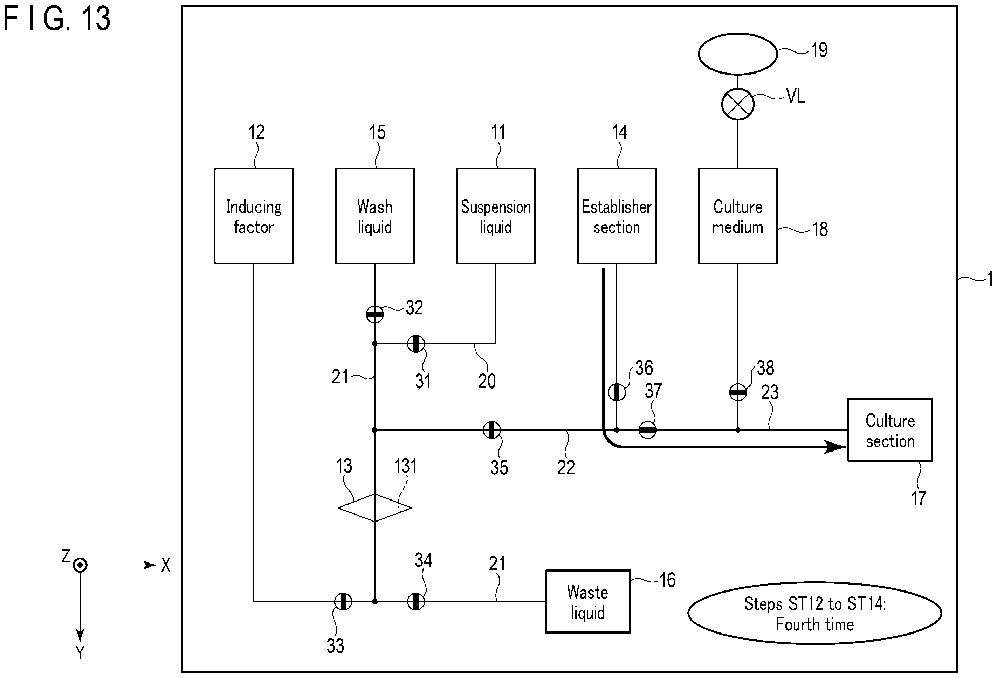

In the cell production cartridge 1, the pin members 33, 35, and 37 are manipulated by the switcher (not shown in the figure) so that the tip positions of the respective pin members 33 and 35 move down to close the corresponding portions of the tube structure 20 and, subsequently, the tip position of the pin member 37 moves up to open the corresponding portion of the tube structure 20 as shown in FIG. 13. This causes a flow channel switchover in the cell production cartridge 1 by which the establisher section 14 is placed in communication with the culture section 17 via the flow channels 22 and 23 (step ST12).

The liquid sending mechanism 4 applies energy to the establisher section 14 by the applicable first mechanism 41 pushing the piston of the establisher section 14 via the corresponding diaphragm 3D (step ST13).

According to the applied energy, the establisher section 14 sends out the established iPS cells through the path after the switchover (step ST14). The establisher section 14 here discharges a suspension liquid containing the iPS cells to the second flow channel 22. The discharged iPS cell-containing suspension liquid moves to the culture section 17.

### (Step ST16)

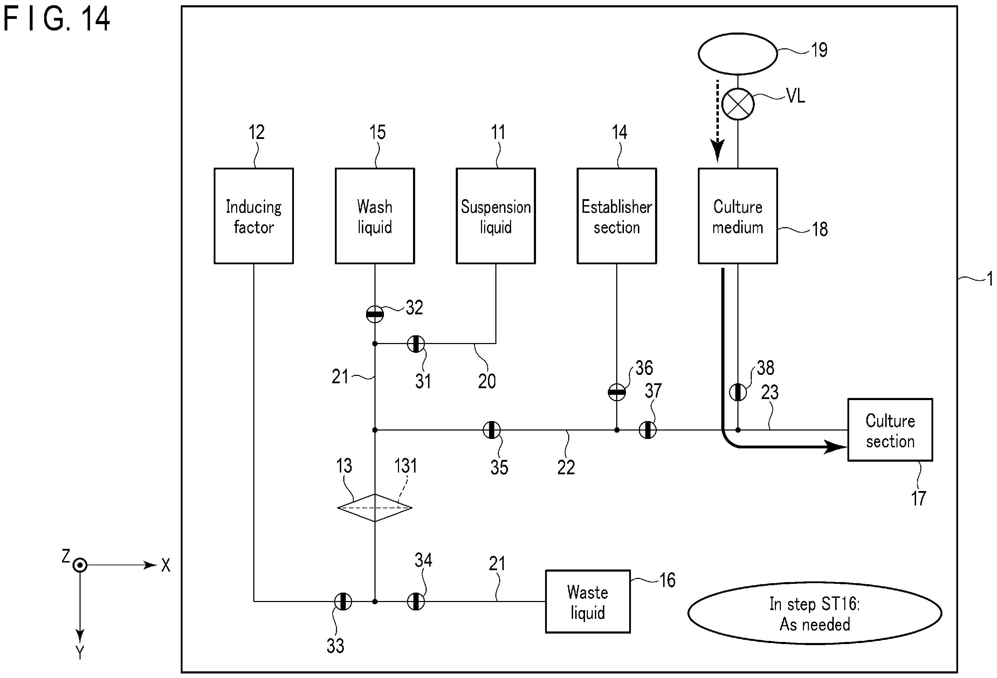

The culturing apparatus 5 adjusts the environment of the culture section 17 in the cell production cartridge 1 to a room temperature of approximately 37 °C. The culture section 17 cultures the iPS cells that have been moved there. Here, the controller controls the switcher and the liquid sending mechanism 4 as needed so that the culture medium is fed from the culture medium feeder 18 to the culture section 17 in the cell production cartridge 1. For example, in the cell production cartridge 1, the pin members 36 to 38 are manipulated by the switcher (not shown in the figure) so that the tip positions of the respective pin members 36 and 37 move down to close the corresponding portions of the tube structure 20 and, subsequently, the tip position of the pin member 38 moves up to open the corresponding portion of the tube structure 20 as shown in FIG. 14. This causes a flow channel switchover in the cell production cartridge 1 by which the culture medium feeder 18 is placed in communication with the culture section 17 via the third flow channel 23. Also for example, in the cell production cartridge 1, the handle 39 is operated by the switcher (not shown in the figure) so that the valve VL is opened. This places the balloon 19 in communication with the culture medium feeder 18 via the valve VL in the cell production cartridge 1.

The liquid sending mechanism 4 applies energy to the culture medium feeder 18 by the second mechanism 42 pushing the balloon 19.

According to the applied energy, the culture medium feeder 18 sends out the culture medium through the path after the switchover. The culture medium feeder 18 here discharges the culture medium to the third flow channel 23. The culture medium discharged to the third flow channel 23 moves to the culture section 17. Thereafter, upon finishing the culturing, the cell production process is ended.

As described above, according to an embodiment, the cell production cartridge 1 includes flow channels and a container for accommodating the flow channels. The flow channels may be flow channels of a closed system. The container may be a container of a closed system. The container includes one or more energy transmission members for receiving, from outside the container, energy for sending and delivering liquids within the flow channels. Each energy transmission member is located at the boundary between the inside and the outside of the container. As such, even in the event that the closed-system flow channels are damaged to let the liquid spill, the closed-system container does not allow such a spilled liquid to leak out. Accordingly, safety can be secured without necessitating a facility investment associated with the risk of liquid leakage. To be more specific, according to an embodiment, it is possible to locally secure safety in the cell production cartridge 1, rather than in a global facility such as a safety cabinet or a laboratory. Also according to an embodiment, once the cell production cartridge 1 is positioned and arranged with the culturing apparatus 5, etc., the accommodated closed-system flow channels and the energy transmission members for sending liquids are also collectively positioned and arranged, and therefore, an enhanced operability can be obtained. In other words, according to an embodiment which adopts a double-closed architecture constituted by the closed-system flow channels and the closed-system container for accommodating the flow channels, safety and operability can be realized at the same time. Since the associated facility investment can be omitted while improving operability, the embodiment allows for high-quality and low-cost cell production. Moreover, automation of steps that require user skills, such as a step for setting the cell production cartridge 1 in an automation mechanism for sending liquids, a step for conducting flow channel switchover, and a step for culturing cells can guarantee a constant quality regardless of user skills.

Also according to an embodiment, the flow channels include one or more at least partly movable reservoirs for storing liquids for use in cell production. Each energy transmission member transfers a driving force corresponding to energy to at least a part of the applicable reservoir. Thus, in addition to the effects discussed above, the transfer of a driving force to the reservoir according to the given energy causes discharge of the liquid according to the driving force, thereby enabling the delivery of the liquid within the flow channels.

According to an embodiment, each energy transmission member is a movable member which relays energy to the applicable reservoir in the form of a mechanical action. Thus, in addition to the effects discussed above, the configuration of the movable member exerting a mechanical action corresponding to the given energy entails a small energy loss at the conversion of the energy into the mechanical action, which can save the energy applied to the cell production cartridge 1.

According to an embodiment, the reservoir may be constituted by a syringe. Thus, in addition to the effects discussed above, quantitative and accurate liquid delivery can be realized.

According to an embodiment, the energy transmission member may be a diaphragm 3D. Thus, in addition to the effects discussed above, it is possible to realize the energy transmission member for dealing with relatively small amounts of liquids for delivery, using a simple configuration of a diaphragm which involves a small amount of displacement.

Also according to an embodiment, the energy transmission member may be a balloon 19. Thus, in addition to the effects discussed above, simplified liquid delivery that does not require quantitative work can be realized.

Also according to an embodiment, the container constituting the cell production cartridge 1 includes the container body 2 in which the concave portion for holding the closed-system flow channels are formed, and the lid 3 attached to the container body 2 so as to cover the concave portion. The lid 3 includes the energy transmission members at positions for covering the respective reservoirs in the flow channels. Thus, in addition to the effects discussed above, the reservoirs and the respective energy transmission members can be arranged to face one another only by closing the lid 3 to the container body 2. That is, the reservoirs and the energy transmission members can be easily positioned.

Also according to an embodiment, the pin members 31 to 38 are each held by the container body 2 and the lid 3 so as to be movable in the direction orthogonal to the flow channel and adapted to close the applicable flow channel by elastically deforming a part thereof. Thus, in addition to the effects discussed above, closing of the flow channels can be realized by a simple configuration of causing the pin members 31 to 38 to squash the corresponding portions of the tube structure 20.

Also according to an embodiment, the flow channels include a tube structure for permitting the liquids discharged from the reservoirs to flow through it, and connectors for detachably connecting the tube structure to the respective reservoirs. Thus, in addition to the effects discussed above, the reservoirs can be connected to and detached from the reservoirs via the respective connectors in any desired manner.

### (Modification 1)

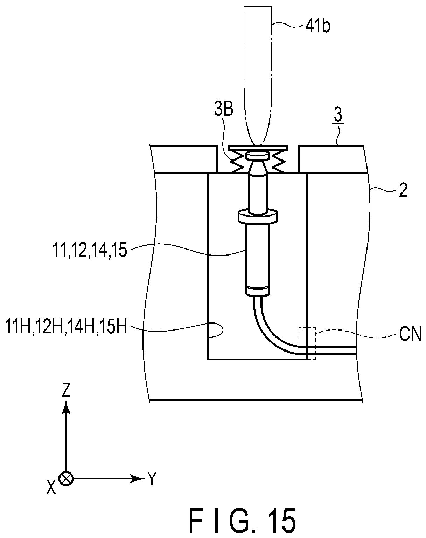

It has been assumed for one embodiment that the cell production cartridge 1 includes one or more diaphragms 3D for receiving energy from the liquid sending mechanism 4, but embodiments are not limited to such a configuration. For example, the cell production cartridge 1 may include a bellows 3B as shown in FIG. 15, instead of each diaphragm 3D. Such a modification can realize, in addition to the effects of the embodiment, the energy transmission member for dealing with relatively large amounts of liquids for delivery, using a simple configuration of a bellows which involves a large amount of displacement. The bellows 3B is another example of the energy transmission member.

### (Modification 2)

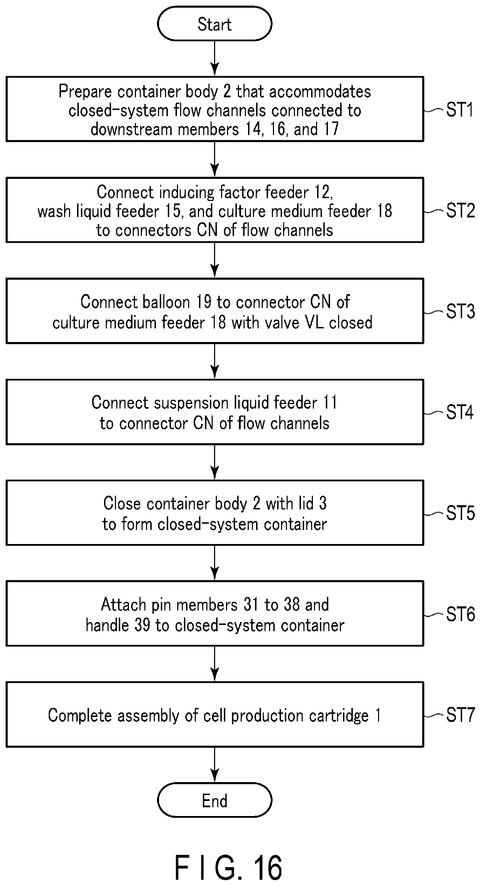

It has been assumed for one embodiment that the cell production cartridge 1 used is a finished product made by a manufacturer or the like, but this does not pose a limitation. For example, the cell production cartridge 1 may be assembled by a user as outlined in FIG. 16. The user, as mentioned in FIG. 16, prepares the container body 2 that accommodates the closed-system flow channels connected to the downstream members such as the establisher section 14, the waste liquid receiver 16, and the culture section 17 (step ST1). In this step ST1, the user may put the closed-system flow channels in a container body 2 made by the manufacturer or the like. As another option, the user may obtain a container body 2 which has been made by the manufacturer or the like and which already accommodates the closed-system flow channels. Next, the user connects the inducing factor feeder 12, the wash liquid feeder 15, and the culture medium feeder 18 to the respective connectors CN of the flow channels (step ST2). The user also connects the balloon 19 filled with air to the upper connector CN of the culture medium feeder 18 while keeping the valve VL in the closed state (step ST3). The user connects the suspension liquid feeder 11 to the corresponding connector CN of the flow channels (step ST4). The closed-system flow channels are thus provided in the container body 2. The user then closes the container body 2 with the lid 3. This forms the closed-system container in which the closed-system flow channels are provided (step ST5). Subsequently, the user attaches the pin members 31 to 38 and the handle 39 to the closed-system container (step ST6). The assembly of the cell production cartridge 1 is thus complete (step ST7). According to such a modification, it is possible to obtain, in addition to the effects of the embodiment, an effect of allowing the user to adopt the suspension liquid feeder 11, the wash liquid feeder 15, the inducing factor feeder 12, and the culture medium feeder 18 each containing a desired liquid.

### (Modification 3)

It has been assumed for one embodiment that the suspension liquid feeder 11, the inducing factor feeder 12, the establisher section 14, and the wash liquid feeder 15 are each constituted by a syringe and a piston, but embodiments are not limited to such a configuration. For example, the suspension liquid feeder 11, the inducing factor feeder 12, the establisher section 14, and the wash liquid feeder 15 may each be a flexible container to which a suitable balloon 19 is connected as appropriate. One preferred example of the flexible container is a flexible bag made of plastic or vinyl. Such a modification can also provide the same effects and advantages as described for the embodiment.

### (Modification 4)

It has been assumed for one embodiment that the culture medium feeder 18 is constituted by a vinyl or a plastic bag connected with the balloon 19, but embodiments are not limited to such a configuration. For example, the culture medium feeder 18 may be constituted by a syringe and a piston and separated from the balloon 19. Such a modification can also provide the same effects and advantages as described for the embodiment.

### (Modification 5)

It has been assumed for one embodiment that the flow channels are closed by causing the pin members 31 to 38 to squash the portions of the tube structure 20, but embodiments are not limited to such a configuration. For example, one or more electromagnetic valves may be connected to the tube structure 20 so as to open and close the flow channels according to the control of each electromagnetic valve. Such a modification can also provide the same effects and advantages as described for the embodiment.

### (Modification 6)

It has been assumed for one embodiment that the flow channel switchover is conducted by causing a given one or a given combination of pin members from the pin members 31 to 38 to squash the corresponding portion or portions of the tube structure 20, but embodiments are not limited to such a configuration. For example, instead of the pin members 31 to 38 arranged above the portions of the tube structure 20, three-way valves may be provided at the respective branch parts of the tube structure 20 so as to conduct the flow channel switchover with these three-way valves. Such a modification can also provide the same effects and advantages as described for the embodiment.

### (Modification 7)

It has been assumed for one embodiment that the cell production cartridge 1 receives kinetic energy from the liquid sending mechanism 4 as the energy for sending and delivering the liquids within the flow channels, but embodiments are not limited to such a configuration. For example, the cell production cartridge 1 may receive any one or combination of potential energy, thermal energy, electric energy, optical energy, and chemical energy from the liquid sending mechanism 4 as the energy for sending and delivering the liquids within the flow channels. The cell production cartridge 1 here may be provided in advance with a mechanism for creating a pressure gradient in the flow channels using the received energy, so that the liquid can be sent and delivered according to the received energy.

### (Modification 8)

It has been assumed for one embodiment that the cell production cartridge 1 has a shape of a horizontally long cuboid constituted by six quadrilateral surfaces, wherein the top and bottom surfaces are each larger than any of the remaining four surfaces, but this does not pose a limitation. For example, the cell production cartridge 1 may have a shape of a vertically long cuboid constituted by six quadrilateral surfaces, wherein two opposing side surfaces are each larger than any of the remaining four surfaces. Such a modification can also provide the same effects and advantages as described for the embodiment.

### (Modification 9)

It has been assumed for one embodiment that the cell production cartridge 1 has a layout capable of accommodating only one set of the closed-system flow channels, but this does not pose a limitation. For example, the cell production cartridge 1 may have a layout capable of accommodating two sets of the closed-system flow channels. The container body 2 of the cell production cartridge 1 here may have a layout which includes a concave portion for use by both of the flow channel sets, a concave portion for use by only one of the flow channel sets, and a concave portion for use by only the other one of the flow channel sets. Likewise, the cell production cartridge 1 may have a layout capable of accommodating three or more sets of the closed-system flow channels. According to such a modification, it is possible to enhance the broad utility of the cell production cartridge 1 in addition to the effects of the embodiment.

### (Modification 10)

It has been assumed for one embodiment that the cell production cartridge 1 has a double-closed architecture constituted by the closed-system flow channels and the closed-system container for accommodating the flow channels, but embodiments are not limited to such a configuration. For example, the cell production cartridge 1 may adopt a triple-closed architecture which further has a container accommodating the double-closed architecture. The triple-closed architecture is not a limitation, and the cell production cartridge 1 may instead adopt a multi-closed architecture which uses four or more closed systems. According to such a modification, it is possible to secure and even enhance the safety in addition to the effects of the embodiment, since the closed-system flow channels can be protected against, for example, a shock that occurs in the event of drop of the cell production cartridge 1.

### (Modification 11)

It has been assumed for one embodiment that the lid 3 is transparent in its entirety, but embodiments are not limited to such a configuration. For example, the lid 3 may be entirely opaque or may be transparent in specific regions. The lid 3 may instead or additionally adopt a configuration which permits one or more light-shielding filters to be freely attached or detached so as to shield the transparent regions as needed. This modification thus enables storage of a liquid which needs to be protected from light, culture of cells in a darkened environment, and so on.

### (Modification 12)

It has been assumed for one embodiment that the container body 2 is opaque in its entirety, but embodiments are not limited to such a configuration. For example, the container body 2 may be transparent in its entirety or in specific regions. The container body 2, if formed to be opaque, may adopt a configuration which permits one or more light-shielding filters to be freely attached to or detached from a given surface or surfaces, or only a given region or regions in the surfaces, so as to shield the transparent regions as needed. This modification thus enables storage of a liquid which needs to be protected from light, culture of cells in a darkened environment, and so on.

According to at least one embodiment described above, safety can be secured without necessitating a facility investment associated with the risk of liquid leakage.

While certain embodiments have been described, these embodiments have been presented by way of example only, and are not intended to limit the scope of the inventions. Indeed, the novel embodiments described herein may be embodied in a variety of other forms. Furthermore, various omissions, substitutions, and changes in the form of the embodiments may be made without departing from the spirit of the inventions. The accompanying claims and their equivalents are intended to cover such forms or modifications as would fall within the scope and spirit of the inventions.

## Claims

1. A cell production cartridge comprising a flow channel and a container for accommodating the flow channel, wherein
the container comprises an energy transmission member for receiving, from outside the container, energy for sending a liquid within the flow channel,
the energy transmission member being located at a boundary between an inside and an outside of the container.

2. The cell production cartridge according to claim 1, wherein
the flow channel comprises a reservoir for storing a liquid for use in cell production, at least a part of the reservoir being movable, and
the energy transmission member is configured to transfer a driving force corresponding to the energy to the at least a part of the reservoir.

3. The cell production cartridge according to claim 2, wherein the energy transmission member is movable and configured to relay the energy to the reservoir in a form of a mechanical action.

4. The cell production cartridge according to claim 2, wherein the reservoir comprises a syringe.

5. The cell production cartridge according to any one of claims 2 to 4, wherein the energy transmission member comprises a diaphragm.

6. The cell production cartridge according to any one of claims 2 to 4, wherein the energy transmission member comprises a bellows.

7. The cell production cartridge according to any one of claims 2 to 4, wherein the energy transmission member comprises a balloon.

8. The cell production cartridge according to any one of claims 2 to 7, wherein the container comprises
a container body in which a concave portion for holding the flow channel is formed, and
a lid attached to the container body so as to cover the concave portion,
wherein the lid comprises the energy transmission member at a position for covering the reservoir in the flow channel.

9. The cell production cartridge according to claim 8, further comprising a closing member held by the container body and the lid so as to be movable in a direction orthogonal to the flow channel, the closing member configured to close the flow channel by elastically deforming a part of the flow channel.

10. The cell production cartridge according to any one of claims 2 to 9, wherein the flow channel further comprises a tube structure for permitting the liquid discharged from the reservoir to flow through the tube structure, and a connector for detachably connecting the tube structure to the reservoir.

11. The cell production cartridge according to any one of claims 1 to 10, wherein the flow channel is a closed-system flow channel.

12. The cell production cartridge according to any one of claims 1 to 11, wherein the container is a closed-system container.

13. A system comprising:
the cell production cartridge according to any one of claims 1 to 12; and
a liquid sending apparatus configured to apply the energy to the energy transmission member.
